# EUROPEAN PATENT APPLICATION

(11) **EP 2 279 765 A1**
(43) Date of publication of application: **02.02.2011**
(21) Application number: 08748399.6
(22) Date of filing: 18.04.2008
(51) Int. Cl.: A61L 27/36, A61L 27/54, A61F 2/02

(54) **A METHOD FOR PREPARING THE DECELLULARIZED MATRIX**

(30) Priority: 24.03.2008 CN 200810026972
(71) Applicant: Zhongshan Ophthalmic Center, Sun Yat-Sen University, Guangdong 510060 (CN); Guangzhou ZhongDa Medical Equipment Co. Ltd., Guangzhou, Guangdong 510275 (CN)
(72) Inventor: WANG, Zhichong, Guangdong 510060 (CN); CHEN, Dong, Guangdong 510060 (CN); WU, Zheng, Guangdong 510060 (CN)
(74) Representative: Hanna, Peter William Derek
(86) International application number: PCT/CN2008/000797
(87) International publication number: WO 2009/117858

(57) **Abstract**

A method for preparing the decellularized matrix using the phospholipase includes the following steps: pretreating the standby tissue and organ; putting the standby tissue and organ into the solution containing the phospholipase; preparing the decellularized matrix in the control condition; washing the prepared decellularized matrix.

## Description

### FIELD OF THE INVENTION

The present invention relates to the field of tissue engineering, and in particular to a method for preparing the decellularized matrix.

### BACKGROUND OF THE INVENTION

Scaffold biomaterials originating from the decellularized matrix have been successfully used in the preclinical research of animal experiment and the clinical application of human diseases. The decellularized matrix can be correspondingly obtained by removing the heterogeneous or homogeneous and allogeneic cells from various tissues and organs and remaining the complicated structures and functional proteins. Different decellularizing methods will directly affect the obtained components of the ultra-structure of the decellularized matrix and finally affect the reaction of the host versus the decellularized matrix after transplantation.

Methods for preparing the decellularized matrix mainly include: 1. physical method; 2. chemical method; 3. enzyme method. The physical method refers to the method in which the cell is destroyed physically by freezing, high voltage, ultrasound, changing osmotic pressure, etc. The physical method has the mainly defect that the ultra-structure of the extracellular matrix is simultaneously destroyed during the cell is destroyed, and the cracked cell fragments must be cleared away, and this cleaning process will further destroy the ultra-structure of the extracellular matrix. While the chemical method includes the method for cell lysis by using acids, alkalis, surfactants, etc. The enzyme method mainly uses protease (such as parenzyme, neutral proteinase) and nuclease (such as exonuclease, endonuclease) to destroy the cell by hydrolyzing the cell protein and the nucleic acid. The cells can be removed effectively by the protease, but a large amount of the structural protein and the functional protein of the extracellular matrix will be degraded due to the wide existing of the substrate of the parenzyme and neutral proteinase. The selective degradation of the nucleic acid by the nuclease will not cause the destroying of the extracellular matrix, but its molecular weight is too large which may induce immunoreaction easily.

In the tissue engineering, a good scaffold biomaterial should have the following technical characteristics: 1. good biocompatibility; the biomaterial and its degradation products is innocuous, and will not induce rejection reaction, inflammatory reaction, teratogenesis, mutagenesis; 2. the biomaterial has various bio-inductive cell growth factors that is inductive to the growth and propagation of autologous cell; 3. the degradation speed of the scaffold biomaterial matches with the speed of forming the tissue and organ for the cell implanted with the decellularized matrix; after finishing its duty, the scaffold can be completely absorbed or integrated with the new tissue; 4. the shaping may be performed in accordance with the characteristic of the specific tissue to realize the perfect repairing; 5. the material has a suitable toughness and machinability and can form a three dimensional structure to provide a sufficient space for material metabolism of the cells. It has been proved by the existing researches that as the scaffold biomaterials in tissue engineering, the decellularized matrix can be obtained widely and is degradable and has good biocompatibility, especially the material may be designed according to the size and thickness of the tissue, and it is adapted to be used in the repairing and reestablishment of various pathological tissue changes. Therefore, it is a great problem to be resolved in the tissue engineering on how to obtain the above five requirements.

### SUMMARY OF THE INVENTION

It is an object of the present invention to obtain a method for preparing a decellularized matrix. The decellularized matrix prepared by this method has good physical properties and biological functions.

The above object is achieved by the following technical solution:
A method for preparing a decellularized matrix comprises the following steps:
   a. pretreating a standby tissue and organ;
   b. putting the standby tissue and organ into a solution containing the phospholipase, preparing the decellularized matrix through hypoosmotic or isoosmotic solution immersion, wherein the phospholipase has a concentration lower than 10000U/ml; and
   c. washing the decellularized matrix prepared.

In the present invention, the solution containing the phospholipase which is more specifically used for hydrolysis of the phospholipid component in the cell of the standby tissue and organ is used such that it is decellularized without damaging the extracellular matrix. Furthermore, the chemical method can be used additionally to speed up the decellularization reaction of the phospholipase by using one or more surfactants that has minimum effect on the extracellular matrix. The physical method may also be used to speed up the decellularization speed according to different requirement of decellularization in different tissues.

The standby tissue and organ may be a heterogeneous or homogeneous and allogeneic or autologous tissue and organ, for example, the tissue and organ may be skin, heart valve, blood vessel, bladder, ligament, cartilage, or nerve; or may be cornea, sclera, conjunctiva, or any combination thereof.

The pretreatment of the standby tissue and organ is routine washing, disinfection and separation, by using the physiological buffer containing antibiotic. The pretreatment may also include hypoosmotic or isoosmotic solution immersion, and vibration, and ultrasonic processing with an adjusted washing temperature, a proper frequency and intensity.

Preferably, the phospholipase is phospholipase A₁, A₂, B₁, B₂, C, D or any combination thereof.

Preferably, the phospholipase has a concentration lower than 10000U/ml, preferably in a range of 1-1000U/ml. The temperature of the solution containing the phospholipase is in a range of 0-56 °C, and the pH value of the solution containing the phospholipase is in a range of 5-12.

During the hypoosmotic or isoosmotic solution immersion, and vibration, the reaction temperature may be adjusted, and the physical method such as ultrasonic processing with a proper frequency and intensity may be performed so as to accelerate the decellularization of the phospholipase.

Preferably, the surfactant is a surface-active component that can be generated in biological body or human body.

Preferably, the surfactant is polyethylene glycol, TritonX-100, cholate, deoxycholate, chenodeoxycholate, glycocholate, glycochenodeoxycholate, taurocholate, taurochenodeoxycholate, lipopolysaccharide, lipoprotein, lysolecithin or any combination thereof.

Preferably, the washing is routine washing of the decellularized matrix by using physiological buffer solution containing antibiotic.

With the using of the phospholipase in the decellularization of the standby tissue and organ, the present invention has the following advantages:
1. The phospholipase is an enzyme that is highly active and specific for hydrolysis of phosphide ester linkage. The main components of the cell membrane are various phospholipids, and thus the phospholipase is specific for the decellularization, and will not damage the structural protein and the functional protein in the extracellular matrix.
2. The molecular weight of the phospholipase is small, and it is unlikely for the residual minimum amount of the phospholipase to induce the immune response.
3. The phospholipase is more effective to the complete cell membrane than to the cell membrane fragments; thus, the cell lysis of the complete cell membrane is performed prior to that of the cell membrane fragments.
4. The hydrolysis reaction is significantly accelerated by the surfactants. The surfactants themselves have the capability of decellularization, but will destroy the protein component in the extracellular matrix to various extents such that many important functional proteins lose their biological function. However, the surfactant in the present invention will facilitate the hydrolysis of the phospholipase and accelerate the hydrolysis speed. The preferred surfactant of the present invention has the following features: (1) the surfactant can facilitate the decellularization effect of the phospholipase; (2) the surfactant induces less damage to the structural and functional proteins in the extracellular matrix. For example, TritonX-100 and sodium deoxycholate both have a large and rigid polarity gene, whereby it is hard for them to enter the crack on the protein surface, therefore, it induces small effect on the protein quaternary structure of the extracellular matrix; (3) when the surfactant is used, on the condition of improving the activity of the phospholipase and facilitating the hydrolysis reaction of the phospholipase, the concentration of the surfactant is reduced in maximum, and it can be implemented by the skilled person in the art according to the known technical theory; (4) the surfactant originating from biological body or human body is preferable. A trace amount of the residue of this kind of surfactant will not affect the normal physiological function of the human body. Therefore, polyethylene glycol, TritonX-100, cholate, deoxycholate, chenodeoxycholate, glycocholate, glycochenodeoxycholate, taurocholate, taurochenodeoxycholate, lipopolysaccharide, lipoprotein, lysolecithin or any combination thereof is preferred in this invention.
5. To further improve the decellularization effect of the phospholipase, the standby tissue and organ may be processed by physical methods. The physical methods utilized in the present invention mainly include hypoosmotic or isoosmotic solution immersion, vibration, temperature adjustment, and ultrasonic processing at a proper frequency and intensity to improve the decellularization result of the phospholipase.
6. The hydrolysis speed of the phospholipase is different under different pH value, temperature and concentration. Thus, these parameters can be regulated to control the hydrolysis speed of the phospholipase in order to cater for the decellularization requirement of different tissues and organs.

In conclusion, the phospholipase is used for the decellularization treatment of the standby tissue and organ in the present invention. According to different decellularization requirements of different tissues and organs, the phospholipase is mainly used, and further the surfactant is used to improve the hydrolysis speed of the phospholipase, and some physical methods may be further added to improve the decellularization results of the phospholipase. The pH value and temperature of the reaction system and the concentration of the phospholipase can be regulated at any moment so as to flexibly control the decellularization process and speed of the phospholipase. With the method of this invention, a decellularized matrix with good physical properties and biological functions can be obtained, which is not only a great breakthrough in the tissue engineering but also develop a new way for therapy of the clinical diseases. The principle of the present invention is reliable with simple process and good repeatability of product, and it is very easy for industrialization.

### BRIEF DESCRIPTION OF THE DRAWINGS

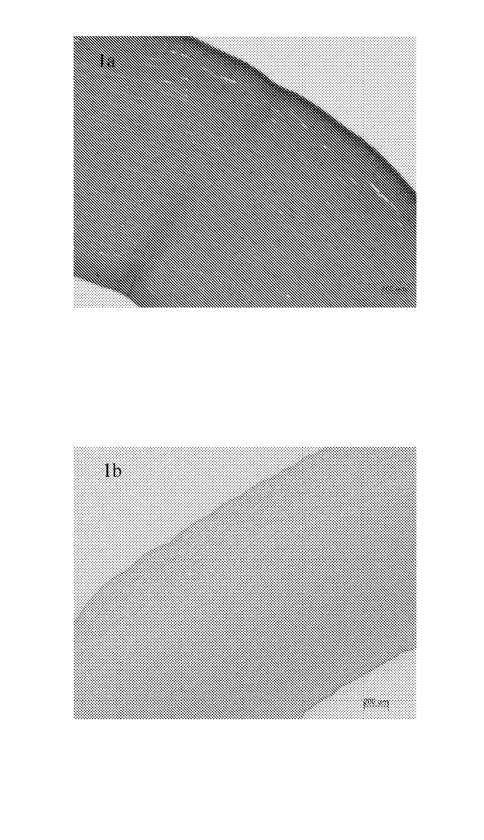

Fig. 1 a is a photograph of a fresh porcine cornea tissue stained by hematoxylin and eosin (HE);
Fig. 1b is a photograph of a decellularized matrix of porcine cornea stained by hematoxylin and eosin;

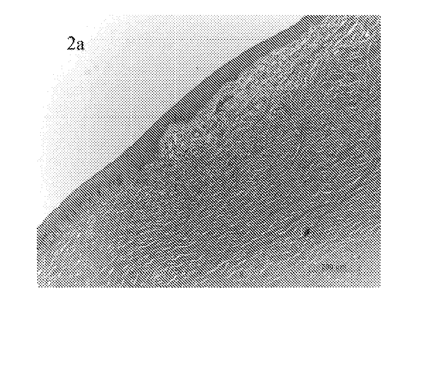

Fig. 2a is a photograph of a fresh porcine cornea limbus stained by hematoxylin and eosin;

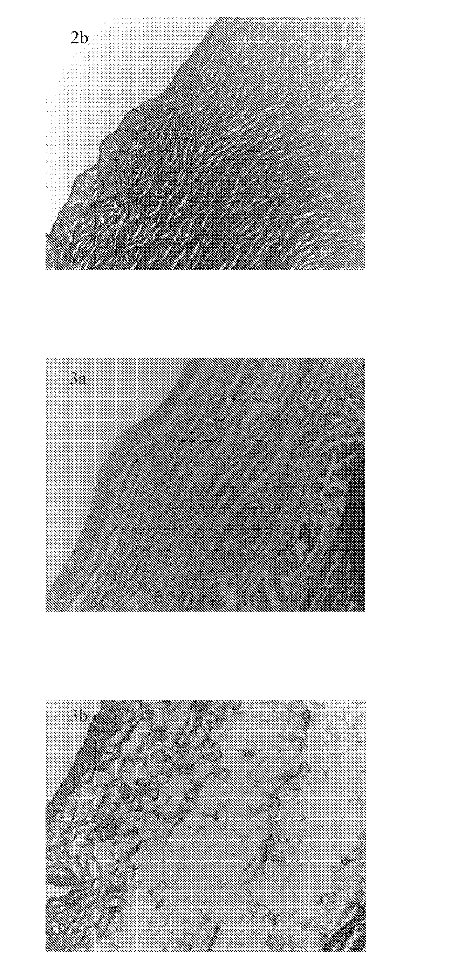

Fig. 2b is a photograph of a decellularized matrix of porcine cornea limbus stained by hematoxylin and eosin;
Fig. 3a is a photograph of a fresh porcine conjunctiva tissue stained by hematoxylin and eosin;
Fig. 3b is a photograph of a decellularized matrix of porcine conjunctiva stained by hematoxylin and eosin;

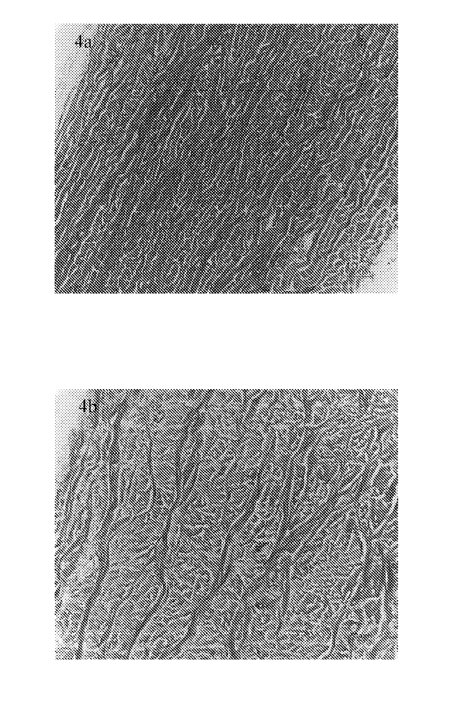

Fig. 4a is a photograph of a fresh porcine sclera tissue stained by hematoxylin and eosin;
Fig. 4b is a photograph of a decellularized matrix of porcine sclera stained by hematoxylin and eosin;

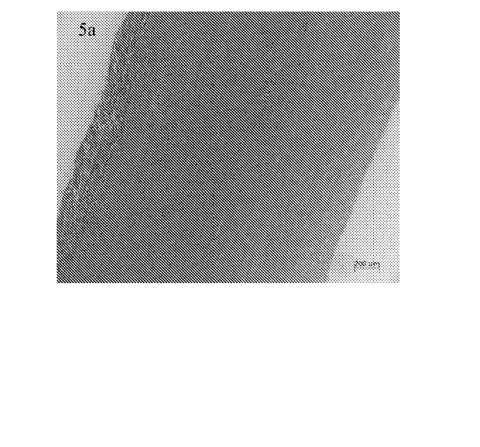

Fig. 5a is a photograph of a porcine aorta tissue stained by hematoxylin and eosin;

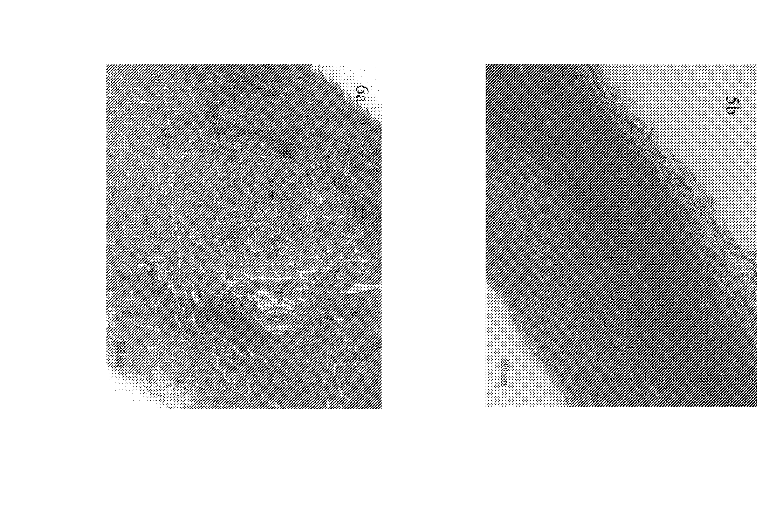

Fig. 5b is a photograph of a decellularized matrix of porcine aorta stained by hematoxylin and eosin;
Fig. 6a is a photograph of a porcine skin tissue stained by hematoxylin and eosin;

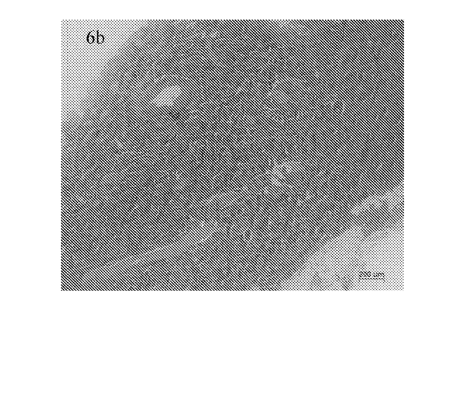

Fig. 6b is a photograph of a decellularized matrix of porcine skin stained by hematoxylin and eosin;

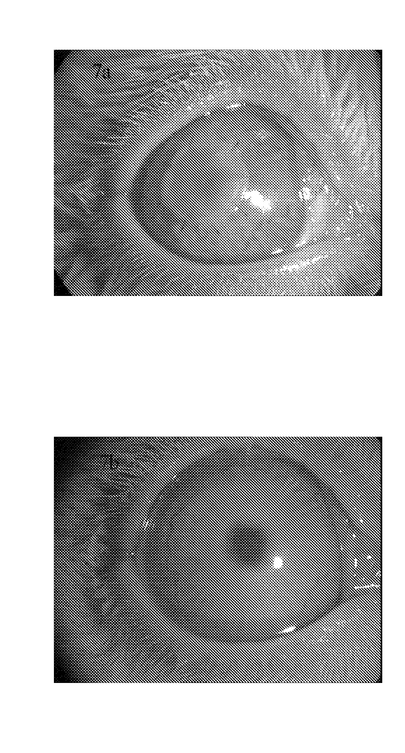

Fig. 7a is a photograph of a rabbit eye 1 day after the lamellar corneal keratoplasty using the decellularized matrix of porcine cornea as donor;
Fig. 7b is a photograph of a rabbit eye 80 days after the lamellar corneal keratoplasty using the decellularized matrix of porcine cornea as donor.

### DETAILED DESCRIPTION OF THE PREFERRED EMBODIMENT

a. Pretreatment of the standby tissue and organ: the standby tissue and organ is taken out at the room temperature according to the routine aseptic operation fundamentals, and preprocessed in different ways according to different tissues and organs. The pretreatment mainly includes the routine aseptic taking of materials, the hypoosmotic or isoosmotic solution immersion, or the ultrasonic processing at a proper frequency and intensity. The purpose for the pretreatment is to enable the phospholipase to be better absorbed into the tissue and to improve the decellularization effect of the phospholipase.

b. The standby tissue and organ is added into the solution containing the phospholipase. According to different tissues and organs, the phospholipase may include phospholipase A₁, A₂ , B₁, B₂, C , D or any combination thereof. The concentration of the phospholipase is lower than 10000U/ml, and preferably in a range of 1-1000U/ml. The temperature of the solution containing the phospholipase is controlled in a range of 0-56°C, and the pH value is in a range of 5 to 12. The decellularization speed of the phospholipase can be increased or reduced by correspondingly changing the types of the phospholipase, the concentration of the phospholipase, the temperature of the solution, the pH value, etc.

According to different tissues and organs and different requirements to the quality of the decellularized matrix, one or more of the surfactants can be added into the solution containing the phospholipase to facilitate the decellularization speed without damaging the extracellular structure and the functional protein. The surfactant originating from biological body or human body is preferable. A trace amount of the residue of this kind of surfactant will not affect the normal physiological function of the human body. For example, polyethylene glycol, TritonX-100, cholate, deoxycholate, chenodeoxycholate, glycocholate, glycochenodeoxycholate, taurocholate, taurochenodeoxycholate, lipopolysaccharide, lipoprotein, lysolecithin or any combination thereof may be used as the surfactant.

To further improve the decellularization result of the phospholipase, the standby tissue and organ may be processed by physical methods. The physical methods utilized in the present invention mainly include hypoosmotic or isoosmotic solution immersion, vibration, temperature adjustment, and ultrasonic processing at a proper frequency and intensity so as to assist in the decellularization of the phospholipase.

c. The decellularized matrix prepared is washed in routine manner by using the physiological buffer solution containing antibiotic. The washing manner, times and duration may be different according to different decellularized matrix prepared. However, the purpose of this is to minimize the residue amount of the decellularization agent. For the phospholipase and the surfactant is harmless in trace amount, the technical requirement of this step is greatly reduced.

### Embodiment 1

The purpose of this embodiment is to prepare the decellularized matrix of porcine cornea by using the porcine cornea. (note: the phospholipase solution may contain the phospholipase A₁, A₂, B₁, B₂, C, D or any combination thereof. The surfactant may be cholate, deoxycholate, chenodeoxycholate, glycocholate, glycochenodeoxycholate, taurocholate, taurochenodeoxycholate, lipopolysaccharide, lipoprotein, lysolecithin or any combination thereof, and also may be polyethylene glycol or TritonX-100, they have the following identical results.)
1. the fresh porcine cornea is taken out by using a 10.0mm trephine at the room temperature according to the routine aseptic operation fundamentals. The porcine cornea is then immersed in the carbonate buffer solution containing antibiotic (100U/ml of penicillin G, 100µg/ml of streptomycin sulfate) for 2-5 times, each time for 2-10 minutes. 2. the porcine cornea is disposed in 10ml of aseptic pure water, and immersed in the water bath at 37°C for duration of 10-60 minutes.
3. the porcine cornea is disposed in the 10ml of aseptic phospholipase solution I, and then vibrated in the water bath at 37°C for duration of 3 to 10 hours. (The aseptic phospholipase solution I is prepared by using the carbonate buffer solution, the pH value is in a range of 8 to 12, wherein the phospholipase A₁+A₂ =50-500U/ml, and the mass percentage concentration of the surfactant sodium deoxycholate is 0.01-1%)
4. the porcine cornea is taken out and washed by using the carbonate buffer solution for 1 to 3 times.
5. the porcine cornea is disposed in 10ml of the aseptic phospholipase solution II, and then vibrated in the water bath at 37°C for duration of 1 to 5 hours. (The aseptic phospholipase solution II is prepared by using the carbonate buffer solution, the pH value is in a range of 5 to 8, wherein the phospholipase A₁+A₂ =50-500U/ml.)
6. the porcine cornea is disposed in 50ml of the carbonate buffer solution containing antibiotic (100U/ml of penicillin G, 100µg/ml of streptomycin sulfate), and then vibrated and washed in the water bath at 37°C for 3 to 6 times, each time for 10 to 30 minutes.
7. the decellularized matrix of porcine cornea prepared is preserved at 4°C under aseptic condition.

### Embodiment 2

The purpose of this embodiment is to prepare the decellularized matrix of porcine cornea limbus by using the porcine cornea limbus. (note: the phospholipase solution may contain the phospholipase A₁, A₂, B₁, B₂, C, D or any combination thereof, and they have the following identical results.)
1. the tissue with the area 2mm outside and inside the fresh porcine cornea limbus is taken out at the room temperature according to the routine aseptic operation fundamentals. The porcine cornea limbus tissue is then immersed in the carbonate buffer solution containing antibiotic (100U/ml of penicillin G, 100pg/ml of streptomycin sulfate) for 2-5 times, each time for 2-10 minutes.
2. the porcine cornea limbus tissue is disposed in 10ml of aseptic pure water, and immersed in the water bath at 4°C for duration of 10-60 minutes.
3. the porcine cornea limbus tissue is disposed in 10ml of the aseptic phospholipase solution, and then vibrated in the water bath at 4°C for duration of 6 to 24 hours. (The aseptic phospholipase solution is prepared by using the carbonate buffer solution, the pH value is in a range of 8 to 12, wherein the phospholipase A₁+A₂ =50-500U/ml)
4. the porcine cornea limbus tissue is disposed in 50ml of the carbonate buffer solution containing antibiotic (100U/ml of penicillin G, 100µg/ml of streptomycin sulfate), and then vibrated and washed in the water bath at 25°C for 3 to 6 times, each time for 10 to 30 minutes.
5. the decellularized matrix of porcine cornea limbus prepared is preserved at 4°C under aseptic condition.

### Embodiment 3

The purpose of this embodiment is to prepare the decellularized matrix of porcine conjunctiva by using the porcine conjunctiva. (note: the phospholipase solution may contain the phospholipase A₁, A₂, B₁, B₂, C, D or any combination thereof. The surfactant may be a surface-active component that can be generated in biological body or human body, for example cholate, deoxycholate, chenodeoxycholate, glycocholate, glycochenodeoxycholate, taurocholate, taurochenodeoxycholate, lipopolysaccharide, lipoprotein, lysolecithin or any combination thereof, and also may be polyethylene glycol or TritonX-100, and they have the following identical results.)
1. a fresh porcine conjunctiva tissue with the area of 1X1 cm is taken out at the room temperature according to the routine aseptic operation fundamentals. The porcine conjunctiva tissue is then immersed in the carbonate buffer solution containing antibiotic (100U/ml of penicillin G, 100µg/ml of streptomycin sulfate) for 2-5 times, each time for 2-10 minutes.
2. the porcine conjunctiva tissue is disposed in 10ml of aseptic pure water, and immersed in the water bath at 10°C for duration of 10-60 minutes.
3. the porcine conjunctiva tissue is disposed in 10ml of the aseptic phospholipase solution, and then vibrated in the water bath at 10°C for duration of 6 to 24 hours. (The aseptic phospholipase solution is prepared by using the carbonate buffer solution, the pH value is in a range of 8 to 12, wherein the phospholipase A₂ =10-300U/ml, and the mass percentage concentration of the surfactant sodium taurocholate is 0.1-5%)
4. the porcine conjunctiva tissue is disposed in 50ml of the carbonate buffer solution containing antibiotic (100U/ml of penicillin G, 100µg/ml of streptomycin sulfate), and then vibrated and washed in the water bath at 10°C for 3 to 6 times, each time for 10 to 30 minutes.
5. the decellularized matrix of porcine conjunctiva tissue prepared is preserved at 4°C under aseptic condition.

### Embodiment 4

The purpose of this embodiment is to prepare the decellularized matrix of porcine sclera by using the porcine sclera. (note: the phospholipase solution may contain the phospholipase A₁, A₂, B₁, B₂, C, D or any combination thereof. The surfactant may be a surface-active component that can be generated in biological body or human body, for example cholate, deoxycholate, chenodeoxycholate, glycocholate, glycochenodeoxycholate, taurocholate, taurochenodeoxycholate, lipopolysaccharide, lipoprotein, lysolecithin or any combination thereof, and also may be polyethylene glycol or TritonX-100, and they have the following identical results.)
1. the fresh porcine sclera tissue with the area of 1X1 cm is taken out at the room temperature according to the routine aseptic operation fundamentals. The porcine sclera tissue is then immersed in the carbonate buffer solution containing antibiotic (100U/ml of penicillin G, 100µg/ml of streptomycin sulfate) for 2-5 times, each time for 2-10 minutes.
2. the porcine sclera tissue is disposed in 10ml of aseptic pure water, and immersed in the water bath at 37°C for duration of 3-12 hours.
3. the porcine sclera tissue is disposed in 10ml of the aseptic phospholipase solution I, and then vibrated in the water bath at37°C for duration of 6 to 24 hours. (The aseptic phospholipase solution I is prepared by using the carbonate buffer solution, the pH value is in a range of 8 to 12, wherein the phospholipase A₁+A₂ =50-500U/ml, the phospholipase B₁+B₂ =50-500U/ml, and the mass percentage concentration of the surfactant sodium deoxycholate is 0.01-0.5%, and the mass percentage concentration of the sodium taurocholate is 0.1-1%)
4. the porcine sclera tissue is taken out and washed by using the carbonate buffer solution for 1 to 3 times.
5. the porcine sclera tissue is disposed in 10ml of the aseptic phospholipase solution II, and then vibrated in the water bath at 37°C for duration of 1 to 6 hours. (The aseptic phospholipase solution II is prepared by using the carbonate buffer solution, the pH value is in a range of 5 to 8, wherein the phospholipase A₁+A₂ =50-500U/ml, and the phospholipase B₁+B₂ =50-500U/ml)
6. the porcine sclera tissue is disposed in 50ml of the carbonate buffer solution containing antibiotic, and then vibrated and washed in the water bath at 40°C for 3 to 6 times, each time for 10 to 30 minutes.
7. the decellularized matrix of porcine sclera tissue prepared is preserved at 4°C under aseptic condition.

### Embodiment 5

The purpose of this embodiment is to prepare the decellularized matrix of porcine thoracic aorta by using the porcine thoracic aorta. (note: the phospholipase solution may contain the phospholipase A₁, A₂, B₁, B₂, C, D or any combination thereof. The surfactant may be a surface-active component that can be generated in biological body or human body, for example cholate, deoxycholate, chenodeoxycholate, glycocholate, glycochenodeoxycholate, taurocholate, taurochenodeoxycholate, lipopolysaccharide, lipoprotein, lysolecithin or any combination thereof, and also may be polyethylene glycol or TritonX-100, and they have the following identical results.)
1. 5 cm of a fresh porcine thoracic aorta is taken out at the room temperature according to the routine aseptic operation fundamentals. The porcine aorta is then immersed in the carbonate buffer solution containing antibiotic (100U/ml of penicillin G, 100µg/ml of streptomycin sulfate) for 2-5 times, each time for 2-10 minutes.
2. the porcine thoracic aorta is disposed in 50ml of aseptic pure water, and immersed in the water bath at 25°C for duration of 6-24 hours.
3. the porcine thoracic aorta is disposed in 50ml of the aseptic phospholipase solution , and then vibrated in the water bath at25°C for duration of 6 to 24 hours. (The aseptic phospholipase solution is prepared by using the carbonate buffer solution, the pH value is in a range of 8 to 12, wherein the phospholipase A₁+A₂ =50-500U/ml, the phospholipase B₁+B₂ =50-500U/ml, the concentration of the phospholipase C is 50-500U/ml, and the mass percentage concentration of the surfactant sodium taurocholate is 0.1-1%, and the molar concentration of the lysolecithin is 10-100umol/L)
4. the porcine thoracic aorta is disposed in 50ml of the carbonate buffer solution containing antibiotic (100U/ml of penicillin G, 100µg/ml of streptomycin sulfate), and then vibrated and washed in the water bath at 25°C for 3 to 6 times, each time for 10 to 30 minutes.
5. the decellularized matrix of porcine thoracic aorta prepared is preserved at 4°C under aseptic condition.

### Embodiment 6

The purpose of this embodiment is to prepare the decellularized matrix of porcine skin by using the porcine skin. (note: the phospholipase solution may contain the phospholipase A₁, A₂, B₁, B₂, C, D or any combination thereof. The surfactant may be a surface-active component that can be generated in biological body or human body, for example cholate, deoxycholate, chenodeoxycholate, glycocholate, glycochenodeoxycholate, taurocholate, taurochenodeoxycholate, lipopolysaccharide, lipoprotein, lysolecithin or any combination thereof, and also may be polyethylene glycol or TritonX-100, and they have the following identical results.)
1. an area 3×3cm² of a fresh porcine skin is taken out at the room temperature according to the routine aseptic operation fundamentals. The porcine skin is then immersed in the carbonate buffer solution containing antibiotic (100U/ml of penicillin G, 100µg/ml of streptomycin sulfate) for 2-5 times, each time for 2-10 minutes.
2. the porcine thoracic aorta is disposed in 50ml of aseptic pure water, and immersed in the water bath at 45°C for duration of 12-24 hours.
3. the porcine skin is disposed in 50ml of the aseptic phospholipase solution , and then vibrated in the water bath at 45°C for duration of 6 to 24 hours. (The aseptic phospholipase solution is prepared by using the carbonate buffer solution, the pH value is in a range of 8 to 12, wherein the phospholipase A₁+A₂ =50-500U/ml, the phospholipase B₁+B₂ =50-500U/ml, the concentration of the phospholipase C is 50-500U/ml, the concentration of the phospholipase D is 50-500U/ml, and the mass percentage concentration of the surfactant sodium deoxycholate is 0.01-0.5%, the mass percentage concentration of sodium taurocholate is 0.1-1%, and the molar concentration of the lysolecithin is 10-100umol/L)
4. the porcine skin is disposed in 50ml of the carbonate buffer solution containing antibiotic (100U/ml of penicillin G, 100µg/ml of streptomycin sulfate), and then vibrated and washed in the water bath at 45°C for 3 to 6 times, each time for 10 to 30 minutes.
5. the decellularized matrix of porcine skin prepared is preserved at 4°C under aseptic condition.

### Embodiment 7

Among the decellularized matrixes prepared in the above embodiments, the technical requirement for preparing the ecellularized matrix of cornea is the highest, because it should meet the requirement to the biocompatibility and the mechanical strength, and further the requirement to the transparence of the living tissue. Therefore, this embodiment is to prove the decellularized matrixes obtained in the present invention can meet the requirement of the biocompatibility and the mechanical strength when it is used in xenotranstlantation.

The decellularized matrix of porcine cornea obtained in the Embodiment 1 is used as the donor material (hereinafter referred to as the donor). A health New Zealand rabbit is selected as the receptor for the lamellar keratoplasty. After the general anesthesia by using Ketamine through intramuscular injection, the eye is disinfected in routine manner, and the eyelids are opened by the eye speculum to suture the superior rectus and the inferior retcus. The receptor implant bed is obtained by punching to a deepness of about 1/3 of the cornea at the center of the receptor cornea by using a 6.5mm trephine and separating the corneal anterior lamella. An iris repositor is used to separate the front layer of the donor cornea (200µm). The donor cornea is disposed on the artifical anterior chamber, the donor implant piece is punched out by using the 6.0 trephine and then disposed on the receptor implant bed, and then the interrupted suture is performed for eight stitches by using the 10-0 nylon suture. When finishing the eye operation, the sutures of the superior rectus and the inferior retcus and the eye speculum are removed, and the eye is applied with the compound tobramycin eye drops. After the eye operation, the eye is applied with the compound tobramycin eye drops once a day. Observe the condition of corneal neovascularization, the epithelization condition and the corneal transparence. After the implantation, it is found that the epithelization is 9 days, and 80 days later, the implant piece is completely recovered into transparence without generating corneal eovascularization.

It should be emphasized that the above-described embodiments can be combined freely. Many variations and modifications may be made to the above-described embodiment(s) of the invention without departing substantially from the spirit and principles of the invention. All such modifications and variations are intended to be included herein within the scope of this disclosure and the present invention and protected by the following claims.

## Claims

1. A method for preparing a decellularized matrix, comprising the following steps:
a. pretreating a standby tissue and organ;
b. putting the pretreated standby tissue and organ into a solution containing the phospholipase, preparing the decellularized matrix through hypoosmotic or isoosmotic solution immersion, wherein the phospholipase has a concentration lower than 10000U/ml; and
c. washing the prepared decellularized matrix.

2. The method of claim 1, wherein the phospholipase is phospholipase A₁, A₂, B₁, B₂, C, D or any combination thereof.

3. The method of claim 1, wherein the phospholipase has a concentration in a range of 1-1000U/ml.

4. The method of claim 1, wherein in the step b, the solution containing the phospholipase further contains surfactants.

5. The method of any one of claims 1 to 4, wherein in the step b, vibration or ultrasonic processing is performed during the immersion.

6. The method of any one of claims 1 to 4, wherein in the step b, the solution has a temperature in a range of 0-56°C, with the pH value in a range of 5-12.

7. The method of claim 4, wherein the surfactant is a surface-active component that can be generated in biological body or human body.

8. The method of claim 4, wherein the surfactant is polyethylene glycol, TritonX-100, cholate, deoxycholate, chenodeoxycholate, glycocholate, glycochenodeoxycholate, taurocholate, taurochenodeoxycholate, lipopolysaccharide, lipoprotein, lysolecithin or any combination thereof.

9. The method of claim 1, wherein the standby tissue and organ is a heterogeneous or homogeneous and allogeneic or autologous tissue and organ.

10. The method of claim 9, wherein the tissue and organ is skin, heart valve, blood vessel, bladder, ligament, cartilage, or nerve.

11. The method of claim 9, wherein the tissue and organ is cornea, sclera, conjunctiva, or any combination thereof.

12. The method of claim 1, wherein in the step a, the pretreatment of the standby tissue and organ is routine washing, disinfection and separation; or routine washing, disinfection, separation, and then hypoosmotic or isoosmotic solution immersion, and vibration or ultrasonic processing.

13. The method of claim 1, wherein in the step c, the washing is routine washing of the decellularized matrix by using a physiological buffer containing antibiotic.
